# EUROPEAN PATENT SPECIFICATION

(11) **EP 2 089 716 B1**
(45) Date of publication and mention of the grant of the patent: **22.06.2011**
(21) Application number: 07819698.7
(22) Date of filing: 08.11.2007
(51) Int. Cl.: G01N 33/574

(54) **METHOD FOR THE DIAGNOSIS OF LEUKEMIA**
LEUKÄMIE-DIAGNOSEVERFAHREN
PROCEDE DE DIAGNOSTIC DE LA LEUCEMIE

(30) Priority: 08.11.2006 EP 06023215
(43) Date of publication of application: 19.08.2009
(73) Proprietor: Medizinische Hochschule Hannover, 30625 Hannover (DE)
(72) Inventor: STANULLA, Martin, 30177 Hannover (DE); CARIO, Gunnar, 24105 Kiel (DE)
(74) Representative: Kröncke, Rolf
(86) International application number: PCT/EP2007/009689
(87) International publication number: WO 2008/055682

(56) References cited:
- US-A- 6 001 973
- US-A1- 2006 057 680
- US-A1- 2006 104 945
- CARIO GUNNAR ET AL: "Distinct gene expression profiles determine molecular treatment response in childhood acute lymphoblastic leukemia" BLOOD, vol. 105, no. 2, 15 January 2005 (2005-01-15), pages 821-826, XP002418905 ISSN: 0006-4971
- SEEGER KARL ET AL: "Different cytokine gene expressions in BM samples from initial and relapsed childhood ALL" BLOOD, vol. 96, no. 11 Part 2, 16 November 2000 (2000-11-16), page 167b, XP008074835 & 42ND ANNUAL MEETING OF THE AMERICAN SOCIETY OF HEMATOLOGY; SAN FRANCISCO, CALIFORNIA, USA; DECEMBER 01-05, 2000 ISSN: 0006-4971
- CHIARETTI S ET AL: "Gene expression profile of adult T-cell acute lymphocytic leukemia identifies distinct subsets of patients with different response to therapy and survival" BLOOD, W.B.SAUNDERS COMPANY, ORLANDO, FL, US, vol. 103, no. 7, 1 April 2004 (2004-04-01), pages 2771-2778, XP002363618 ISSN: 0006-4971
- CARIO G ET AL: "High interleukin-15 expression characterizes childhood acute lymphoblastic leukemia with involvement of the CNS" JOURNAL OF CLINICAL ONCOLOGY 20 OCT 2007 UNITED STATES, vol. 25, no. 30, 20 October 2007 (2007-10-20), pages 4813-4820, XP008087196 ISSN: 0732-183X
- CARIO GUNNAR ET AL: "High interleukin 15 expression characterizes childhood acute lymphoblastic leukemia with involvement of the central nervous system." BLOOD, vol. 108, no. 11, Part 1, November 2006 (2006-11), pages 642A-643A, XP008087158 & 48TH ANNUAL MEETING OF THE AMERICAN-SOCIETY-OF-HEMATOLOGY; ORLANDO, FL, USA; DECEMBER 09 -12, 2006 ISSN: 0006-4971

## Description

The present invention relates to a method for the diagnosis of the involvement of the central nervous system in a subject afflicted with leukemia. In particular, the present invention relates to the determination of the relative level or absolute amount of interleukin-15 (IL-15) and/or interleukin-15 receptor alpha chain (IL-15Ralpha) in a sample from a subject afflicted with leukemia and comparing said level and/or amount with the level and/or amount of IL-15 and/or IL-15Ralpha in a control sample. An elevated relative level and/or absolute amount of IL-15 and/or IL-15Ralpha in the sample from a subject afflicted with leukemia relative to the control is indicative of the involvement of the central nervous system in leukemia.

Furthermore, the present invention relates to a method for the stratification of a subject being afflicted with leukemia determining the therapy regimen for the treatment of leukemia comprising the determination of the relative or absolute level of IL-15 and/or IL-15Ralpha in said subject.

### Prior art

The major forms of leukemia are divided into four categories. Myelogenous and lymphocytic/lymphoblastic leukemia each have acute and chronic forms. The terms myelogenous or lymphocytic/lymphoblastic denote the cell type involved. In addition, within each category, distinct leukemias are defined according to a combination of morphology, immunophenotype, and cytogenetic features in addition to clinical symptoms.

Acute leukemia is a clonal disease of hematopoetic stem cells. For example, in the United States more than half of all new leukemias are acute leukemias. Said acute leukemias can be differentiated into acute myelogenous leukemia (AML), also called acute non-lymphocytic leukemia and acute lymphocytic or acute lymphoblastic leukemia (ALL) based on whether hematopetic stem cells have been differentiated along the lymphoid or myeloid lines.

The acute lymphoblastic leukemia is the most frequent type of cancer in childhood amounting 30 % of all cases. About 3.7 of 100.000 children under the age of 15 are suffering from ALL. For example, in Germany this amounts to approximately 600 new cases each year. Successful treatment strategies for childhood acute lymphoblastic leukemia are based on essential therapeutic phases that are consecutively applied over a time period of 2 to 3 years and lead to an overall long-term survival of approximately 80 %. Usually, the therapy includes chemotherapy and irradiation. However, about 20 % of the patients suffer from recurrence which can originate from the central nervous system (CNS) or from the bone marrow of the subjects. The CNS-directed therapy has be become a prerequisite for successful treatment of childhood ALL. Before the introduction of CNS-directed therapy in the 1960's, up to 75 % of children with ALL suffered from diseases recurrence originating from the CNS. Today, acute leukemia, in particular ALL, has the highest propensity to invade the meninges and to cause leukemic CNS disease. Also for other types of leukemia, e.g. the acute myelomonocytic leukemia (AMML), high risk for the development of leukemic CNS disease is described. In addition, also for other types of lymphoid hematopoietic malignancies, like the non Hodgkin's lymphoma entities Burkitt's lymphoma and lymphoblastic lymphoma, invasion of the meninges can be observed.

After applying CNS-directed therapy the high rate of disease recurrence originating from the CNS could be dramatically reduced to less than 5 % through the introduction of cranial irradiation, intrathecal chemotherapy of methotrexate alone or combination with other drugs and systemic application of chemotherapeutics with adequate penetration into the CNS. Also in adult patients with acute leukemia only 5 to 10 % develop recurrent disease with CNS involvement.

On the other hand, patients developing CNS recurrence with leukemia have a poor prognosis outlining the importance of identifying and stratifying the appropriate therapy. Presently, only 3 to 5 % of childhood ALL patients are positive for CNS involvement based on the occurrence of leukemia cells in the cerebrospinal fluid of said patients. As indicated before, after introduction of CNS-directed therapy the rate of recurrence with involvement of the CNS decreased to 4 to 5 % today. However, about 60 % of the patients having recurrence with involvement of CNS are initially diagnosed as being CNS negative.

Thus, an insufficient characterization of the patients with the presently applied methods for diagnosing leukemia, results in high recurrence rates of leukemia with involvement of CNS. Today, the laboratory test used to diagnose CNS involvement in leukemia is the examination of the cerebrospinal fluid (CSF), which is usually obtained by lumbar puncture. CSF cytology positive for malignant cells is the standard method in most clinical series by which malignant CNS involvement is diagnosed. However, as mentioned before, most patients with disease recurrence involving the CNS were initially diagnosed as being CNS-negative.

Today, the intensity of CNS-targeted treatment is adjusted according to the risk of ALL relapse originating from the CNS, the most important risk factor being overt CNS involvement at diagnosis, which is >5 leukocytes/µl CSF in the presence of lymphoblasts. Additional, risk factors include a high initial white blood cell count, pro-B or T-cell immunophenotype, and a traumatic lumbar puncture with identifiable blast cells present at diagnosis. The CNS risk profile is important for the decision on the intensity of CNS-directed therapy, which may differ in the number of intrathecal injections and/or intrathecally applied drugs, as well as in the inclusion of cranial irradiation at different doses.

However, the problem involved in CNS-directed therapy is that at higher intensity of CNS-directed therapy, especially cranial irradiation, long term sequelae may occur (e.g. impaired intellectual and psychomotor functioning; neuroendrocrine system abnormalities and secondary malignancies).

Hence, despite extensive attempts to accurately assess CNS risk at diagnosis and to adjust routine CNS-directed therapy to avoid over- or undertreatment, the majority of relapses with CNS involvement occur in ALL patients that are CNS-negative at diagnosis. This indicates that the current CNS risk stratification strategies are insufficient for a comprehensive characterization of CNS status at diagnosis.

Thus, the problem underlying the present invention is a provision of a more precise CNS risk assessment to improve control of leukemia, like ALL in the CNS, to overcome the problems outlined above, namely, the recurrence of leukemia, like ALL with involvement of CNS in initially CNS-negative ALL patients, and also to avoid overtreatment with potential deleterious long-term effects.

Cario et al., 2005 discloses expression profiles of several markers to predict relapse of ALL.

Seeger et al., 2000 discloses that the level of IL-15 is higher at initial diagnosis of ALL than at the time of relapse, but no follow-up study is performed to show the value of IL-15 in relapse prediction.

Chiaretti et al., 2004 discloses expression profiles of several markers and their comparison to treatment response, as a basis to stratify patients with ALL to determine the treatment regimen.

### Summary of the present invention

The inventors found that interleukin-15 (IL-15) and interleukin-15 receptor alpha chain (IL-15Ralpha) are suitable biological markers for leukemia with involvement of the CNS in a subject afflicted with leukemia, in particular, with acute leukemia.

Hence, the present invention relates to a method for the diagnosis of the involvement of the CNS in a subject afflicted with leukemia comprising determining the relative level and/or absolute amount of IL-15 and/or IL-15Ralpha in a sample from said subject. Further, said level and/or amountof IL-15 and/or IL-15Ralpha is compared with the level and/or amount of IL-15 and/or IL-15Ralpha in a control sample. An elevated level and/or amount of IL-15 and/or IL-lSRalpha in the sample from said subject relative to the control is indicative of the involvement of the CNS in leukemia in said subject.

In another embodiment the present invention relates to a method for the stratification of a subject being afflicted with leukemia to determine the therapy regimen for the treatment of said disease or disorder, e.g. leukemia, comprising determining the relative level and/or absolute amount of IL-15 and/or IL-15Ralpha in a sample from said subject and comparing the level and/or amount of IL-15 and/or IL-15Ralpha to

the level and/or amount of IL-15 and/or IL-15Ralpha in a control sample, with the level and/or amount of IL-15 and/or IL-15Ralpha in samples obtained prior to the begin of the therapy of said subject, or obtained in earlier stages of the regimen from said subject. In preferred embodiments the sample to be used for determining the level and/or amount of IL-15 and/or IL-15Ralpha is obtained from body fluids, in particular, from bone marrow, blood, or CSF.

The level and/or amount of IL-15 and/or IL-15Ralpha may be determined on mRNA or protein level. Preferably, the determination of the relative level and/or absolute amount of IL-15 and/or IL-15Ralpha is performed by real time quantitative polymerase chain reaction analysis after reverse transcription.

In another preferred embodiment the leukemia is childhood leukemia, in particular, childhood acute lymphoblastic leukemia (ALL) or childhood acute myelogenous leukemia (AML).

In another preferred embodiment, the stratification for leukemia therapy is performed based on the results of the first diagnosis of leukemia with involvement of the CNS, during initial treatment of said disease or disorder, like leukemia with or without involvement of the CNS, after the first regimen of treatment of said disease or disorder, like leukemia or during monitoring of leukemia relapse.

### Brief description of the drawings

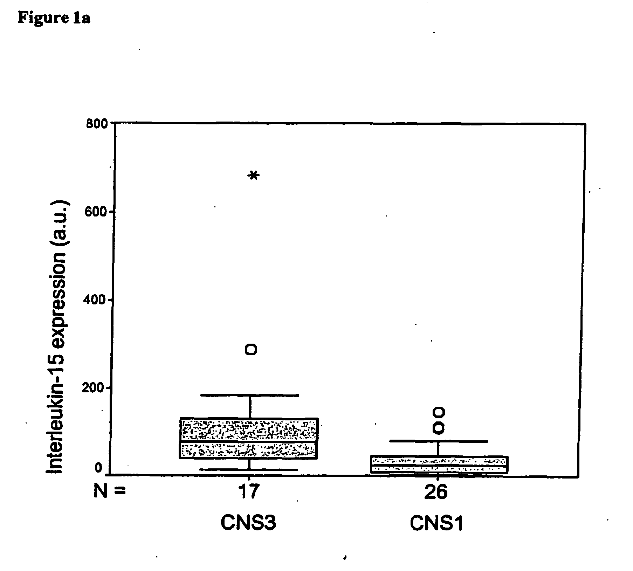

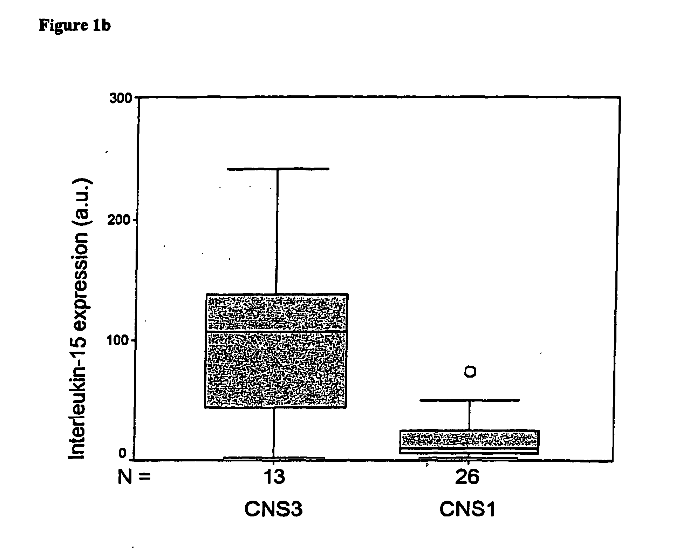

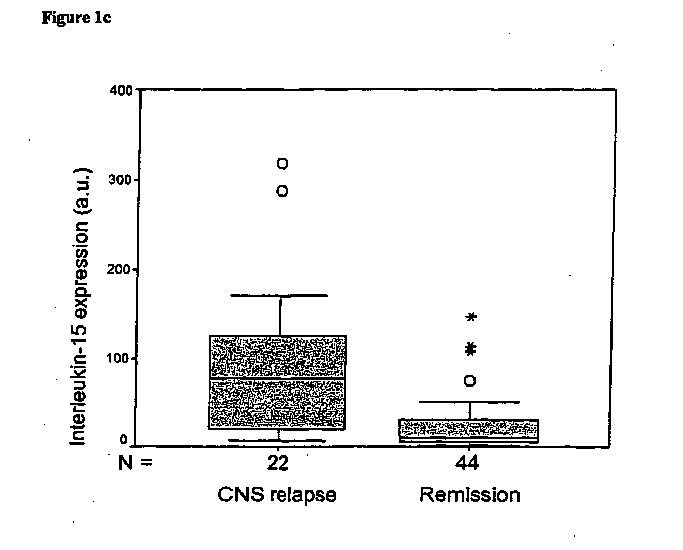

**Figure 1** Box plots demonstrating the positive association between the expression of interleukin-15 (IL-15) and CNS involvement in leukemia: **(A)** Leukemic IL-15 expression at diagnosis in patients with acute lymphoblastic leukemia with (CNS3) and without (CNS1) leukemic involvement of the central nervous system. Data depicted were derived through RQ-PCR after reverse transcription using RNA of leukemic bone marrow cells from those patients included in gene expression analyses using microarays. **(B)** Leukemic IL-15 expression at diagnosis in patients with acute lymphoblastic leukemia with (CNS3) and without (CNS1) leukemic involvement of the central nervous system. Data depicted were derived through RQ-PCR after reverse transcription using RNA of leukemic bone marrow cells from a set of patients independent from those shown in (A). **(C)** Leukemic IL-15 expression at primary diagnosis in patients with acute lymphoblastic leukemia with subsequent relapse involving the CNS compared to patients in long-term remission. Both groups were classified as CNS1 at diagnosis using conventional methods for assessment of CNS status.

The line within the box plots corresponds to the median value, the box length to the interquartile range, and the lines emanating from the box (whiskers) extend to the smallest and largest observations; outliers and extremes are indicated; a.u. = arbitrary units.

### Detailed description of the present invention of said leukemia

The term "leukemia" according to the invention comprises a cancer of blood-forming tissues of the bone marrow. It is characterized by the growth of immature white blood cells. The disease can be categorized based on which blood cells are abnormal. Lymphomas are cancers of lymphoid tissue (lymph nodes, spleen, and other organs of the immune system).

As used herein, the term "subject" or "individual" or "patient" which is used herein interchangeably refers to an individual or a subject or a patient in need of a therapy or suspected to be afflicted with a condition or disease, namely leukemia. Preferably, the subject or individual is a vertebrate even more preferred a mammal, particularly, preferred a human.

The method according to the present invention is preferably conducted by collecting a sample from the subject and measuring in vitro the level and/or amount of IL-15 and/or IL-1 5Ralpha using appropriate means.

The term "control sample" as used herein refers e.g. to a sample of an individual of the same species which individual is not suffering from lymphoma or leukemia with involvement of the CNS. Preferably, the negative samples of the same type as the sample. In addition, the control sample may be a sample from an individual not afflicted with leukemia.

A sample according to the invention is biological material, which has been obtained from an individual. In particular, said term includes material obtained from blood, plasma, tissue, bone marrow or CSF. Particular, preferred the sample is obtained from bone marrow, blood, or CSF.

The expression "level" or "amount" is meant to describe the relative level of said molecules or the absolute amount of said molecules according to the present invention relative to the same molecule in a control sample. Relative means that no distinct amounts such as mole or milligram per litre etc. are stated, but that for example is stated the sample contains more, less or the same amount of a certain molecule as compared to a control sample. The term "more, less or the same amount" in this situation includes also arbitrary units.

With "elevated" or "increased" according to the invention is meant, that the molecule is present in a sample in higher quantities, or amounts or concentration, as compared to another sample, or as compared to a control sample or as compared to a negative control sample or as compared to a reference value, regard as if these measurements are relative or absolute measurements. Preferably, the molecule which is increased is present in a concentration, or quantity or amount which is at least 10 %, at least 20 %, at least 30 %, at least 40 %, at least 50 %, at least 60 %, at least 70 %, at least 80 %, at least 90 %, at least 100 %, at least 200 %, at least 300 %, at least 400 %, at least 500 %, at least 1000 % or at least more than 1000 % above the value to which it is compared. In other words, preferably the molecule is increased at least 2-fold, 3-fold, at least 3-fold, at least 4-fold, at least 5-fold, at least 10-fold above the level and/or amount in the control sample.

With "stratification of a subject" according to the invention is meant to determine the therapy regimen for the treatment of leukemia. In particular, said stratification includes determining whether said subject will benefit from irradiation, in particular, from cranial irradiation, and determining the intensity of CNS-directed therapy. Said stratification allows CNS risk assessment in patients afflicted with leukemia.

The term "involvement of the CNS" refers to a disease state where invasion of the meninges by leukemic cells is present.

Thus, the present invention relates to a method for the diagnosis of the involvement of the CNS in leukemia in a subject. Said method comprises the steps of determining the relative level and/or absolute amount of IL-15 and/or IL-15Ralpha in a sample from said subject and comparing the level and/or amount of IL-15 and/or IL-15Ralpha to the level and/or amount of IL-15 and/or IL-15Ralpha in a control sample wherein the elevated level and/or amount of IL-15 and/or IL-15Ralpha in the sample from said subject relative to the control is indicative of the involvement of the CNS in leukemia in said subject. That is, the elevated expression level of IL-15 or IL-15Ralpha chain at initial diagnosis of leukemia allows to assess the involvement of the CNS in said leukemia.

At present, CNS status at the time of diagnosis, e.g. diagnosis of childhood ALL, is commonly assessed by examination of CSF obtained by lumbar puncture. However, in the brains of children with ALL after autopsy CNS involvement in more than 50 % of the examined specimens was revealed. Further, without targeted CNS therapy more than 50 % of the patients will relapse in the CNS suggesting that CNS involvement is present at diagnosis in the majority of these patients. The use of IL-15 and IL-15Ralpha levels now allows to diagnose leukemia with CNS involvement more precisely and, thus, allow to adjust the toxic CNS-directed anti-leukemia therapy more accurate. The determination of the relative level and/or absolute amount of IL-15 and/or IL-15Ralpha not only allows to initially diagnose leukemia with CNS involvement but also enables stratification of the subjects afflicted with leukemia. Hence, in a further embodiment the present invention relates to a method for the stratification of the subject being afflicted with leukemia to determine the therapy regimen for the treatment of leukemia comprising a) determining the relative level and/or absolute amount of IL-15 and/or IL-15Ralpha in a sample from said subject; and b) comparing the level and/or amount of IL-15 and/or IL-15Ralpha to the level and/or amount of IL-15 and/or IL-15Ralpha in a control sample, with the level and/or amount of IL-15 and/or IL-15Ralpha in samples obtained prior to the begin of the therapy of said subject, or obtained in earlier stages of the regimen from said subject.

That is, determining the relative level and/or absolute amount of II-15 and/or IL-15Ralpha chain in subjects undergoing the first diagnosis of leukemia is indicative for a CNS involvement, thus, requiring adapted CNS treatment in the therapy regimen of leukemia. In addition, IL-15 levels diagnosed during the initial therapy or during follow-up control after initial treatment is indicative for an eventual CNS relapse. Thus, controlling the IL-15 or IL-15 receptor levels allows to stratify whether patients with our without CNS involvement may have a CNS relapse. To conclude, the present invention provides a potential diagnostic tool and method for assessing CNS involvement in leukemia, in particular, in childhood ALL or AML, for protection of CNS relapse and CNS involvement in leukemia when assessing the IL-15 or IL-15Ralpha expression.

The samples may be obtained from bone marrow, CSF, or blood. A sample can also be material indirectly obtained from an individual, such as cells obtained from the individual which have been cultured in vitro, prior to obtain a sample from these in vitro cultured cells. A sample can also be pretreated prior to analysis with the methods of the invention. Such pretreatments for example can be storage of the sample at various temperatures, such as room temperature, 4 °C, 0 °C, -20 °C, -70 °C, -80 °C, or other temperatures or storage on water ice or dry ice, or storage in liquid nitrogen or storage in other solid, liquid or gas media.

Suitable methods to detect the relative level and/or absolute amount of IL-15 or IL-15Ralpha in the samples are well known in the art. For example, suitable methods include suitable mass spectrometry methods, e.g. matrix assisted laser desorption ionisation (MALDI), continuous or pulsed electrospray ionisation (ESI) and related methods such as ionspray or thermospray or massive cluster impact. The ion sources can be matched with detection formats including linear or non-linear reflection time of light (TOF), single or multiple quadrupole, single or multiple magnetic sector, Fourier transformation ion cyclotron resonance (FTICR), ion trap, and combinations thereof. Other mass spectrometic methods suitable are for example fast atom bombardment (FAB), mass spectrometry, surface enhanced laser desorption (ionisation) (SELDI), masssspectrometry, etc.

Further, suitable immunologic methods among other are enzyme link immunoassays (ELISA), sandwich, direct, indirect, or competitive ELISA assays, enzyme link immunospotassays (ELlspot), radio immunoassays (RIA), flow-cytometry assays (FACS), immunohistochemistry, Western Blot, fluorescence resonance energy transfer (FRET) assays, protein chip assays using for example antibodies, antibody fragments, receptor ligands or other agents binding the molecules of the invention.

Among other Northern Blot or Southern Blot hybridization, nucleic acid dot or slot Blot Hybridization, in situ hybridization, nucleic acid chip assays (for example using RNA, DNA or other nucleic acids to specifically capture the nucleic acid of interest), polymerase chain reaction (PCR), reverse transcriptase-PCR (RT-PCR), real time PCR, and in particular, real time quantitative polymerase chain reaction (RQ-PCR) after reverse transcription may be used as molecular biology methods to detect the molecules of the invention.

Further methods, such as nuclear magnetic resonance (NMR), fluorometry, colorimetry, radiometry, luminometry or other spectrometric methods, liquid chromatography, capillary chromatography, thin-layer chromatography, plasmon resonance, one-or-two gel dimensional electroporesis etc. can be used to detect the molecules of the invention.

In another preferred embodiment the level and/or amount of IL15 and/or IL-15Ralpha is determined on mRNA level. Particular preferred the determination is conducted by RQ-PCR, for example applying known real time PCR-methods. Another preferred embodiment relates to the determination of the level and/or amount of IL-15 and/or IL-15Ralpha on protein level. For example, said level may be determined by immunological methods as outlined above using specific IL-15 and/or IL-15Ralpha antibodies.

In another preferred embodiment, the level and/or amount of IL-15 and/or IL-15Ralpha is determined using flow cytometry analysis of cells which are obtained from body fluids of the subjects. Particular preferred the cells are obtained from bone marrow, blood, or CSF.

The control sample to be used in the method of the present invention are samples obtained from individuals not afflicted with leukemia or from individuals afflicted with leukemia but known to have no involvement of the CNS.

In another embodiment, e.g. for stratification of the subject, the "control sample" is a sample obtained before from the same subject, e.g. obtained prior the begin of therapy or at earlier stages of the therapy to monitor the necessity to continue with a respective intensity of CNS treatment. When diagnosing leukemia with involvement of the CNS, the elevated level relative to the control sample is at least 2-fold, 3-fold, 4-fold, preferably at least 5-fold or 10-fold relative to the level and/or amount of IL-15 and/or IL-15Ralpha of the control sample.

The methods according to the present invention are suitable for diagnosing or stratification of a subject afflicted with any kind of leukemia. Thus, lymphoid and myelogenous leukemias of acute and chronic types can be diagnosed independent of the patient's age at diagnosis. Particular preferred the diagnosis is for acute leukemia, in particular, for acute myelogenous leukemia (AML) and acute lymphoblastic/lymphocytic leukemia (ALL) and most preferably in childhood ALL and childhood AML. In addition, the present invention is also suitable for diagnosing CNS involvement of other hematological malignant neoplasias such as lymphomas (e.g., the non-Hodgkin and Hodgkin lymphomas) and can be used for guiding the intensitiy of CNS-directed treatment in these diseases. Similarly, the invention presented herein can be used to diagnose the propensity of solid malignant neoplasias such as carcinomas to involve the CNS (e.g., in the form of metastasis). Also in this latter instance, the invention allows guidance of the intensity of CNS-directed treatment in these diseases.

### Examples

The following examples are provided to illustrate the invention. However, they are not meant to limit the scope of the invention in any way.

### Methods

### Patients

Patients were newly diagnosed with ALL between October 1999 and May 2004 and were aged 1 to 18 years. Informed consent was obtained from patients or legal guardians and the protocol approved by the local ethical committee. Leukemia diagnoses were made according to morphological criteria of the French-American-British (FAB) classification and by immunophenotyping of blast cells according to recommendations of the European Group for the Immunological Characterization of Leukemias (EGIL) in reference laboratories. CNS status was defined as follows: CNS1 (puncture nontraumatic without leukemia blasts after cytocentrifugation) CNS2 (puncture nontraumatic, ≤ 5 white blood cells (WBC)/µL CSF with identifiable blasts), CNS3 (puncture nontraumatic, > 5 WBC/µL CSF with identifiable blasts), traumatic lumbar puncture (TLP)+ (TLP with blasts), and TLP- (TLP without blasts). A TLP was defined as 10 or more erythrocytes per microliter CSF or macroscopially contaminated CSF. In addition to the CNS3 group as defined above, patients with a cerebral mass or patients with cranial nerve palsy in combination with blasts after cytocentrifugation were regarded as having CNS3 disease. Complete remission (CR) was defined as the absence of leukemic blasts in the peripheral blood and cerebrospinal fluid, less than 5 % lymphoblasts in bone marrow aspiration smears, and no evidence of localized disease. Relapse was defined as recurrence of lymphoblasts or localized leukemic infiltrates at any site. Isolated CNS relapse was defined by a CSF with > 5 WBC/µL CSF with identifiable blasts on cytospin preparations with less than 5 % of bone marrow blasts, and no other localized leukemic infiltrates. Combined relapses included those with simultaneous recurrence in the bone marrow and in an extramedullary site.

For gene expression analysis using microarrays, patients with CNS3 status and available diagnostic specimens from trial ALL-BFM 2000 who could be matched to at least one CNS1 control sample according to gender age at diagnosis (between 1 to 10 y; ≥ 10 years), initial WBC count (< 10,000/µl; 10,000 to < 50,000/µl; 50,000 to < 100,000/µl; ≥ 100,000/µl), and immunophenotype (pro B, precursor B, common, T-cell were included). Validation analysis in independent patients with CNS3 status from the same population could only be performed with control individuals matched according to immunophenotype. Patients that were initially CNS-negative (CNS1) with subsequent isolated or combined CNS relapse were compared to CNS1 patients with a minimum follow-up of five years. Bone marrow or peripheral blood specimens were required to contain more than 70 % blasts as assessed morphologically before gradient centrifugation.

Initially nine precursor B-cell ALL patients with CNS3 status who were matched to 18 control subjects with CNS1 status according to gender, age at diagnosis, initial WBC count, and immunophenotype (two control CNS1 patients per one CNS3 patient) were identified. Using the same criteria, eight additional patients with T-cell ALL and CNS3 status were matched to eight controls. Characteristics of these patients are given in Table 1.

### Leukemic cell purification

Leukemic cells from kryopreserved diagnostic ALL specimens were purified with a FACSVantage cell sorter (Becton Dickinson, Heidelberg, Germany) using anti-CD19 and anti-CD10 antibodies (Becton Dickinson).

### RNA isolation, amplification, and reference RNA

Total RNA of the primary set was isolated with Trizol reagent (Invitrogen, Paisley, United Kingdom) and subsequently passed over a Qiagen RNeasy column (Qiagen, Hilden, Germany) for the removal of small fragments. Each of the total RNA samples was linearly amplifies using the MessageAmp aRNA Kit (Ambion, Austin, TX). Total and amplified RNA were quantified and validated for integrity using the Bioanalyzer 2100 (Agilent, Palo Alto, CA). The reference RNA used for all arrays was Universal Human Reference RNA (Stratagene Europe, Amsterdam, The Netherlands) linearly amplified using the same method.

### Gene expression measurements and analysis

Spotted cDNA microarrays that contained more than 43,000 features representing approximately 30,000 genes were used (Stanford Functional Genomics Facility, Stanford, CA). The sample RNA and the reference RNA with different fluorescent dyes (Cy5-deoxyuridine triphosphate (dUTP) and Cy3-dUTP; Amersham Pharmacia Biotech Europe, Freiburg, Germany) and comparatively hybridized them to an array. The fluorescence intensities of Cy5 and Cy3 were measured using a GenePix 4000 scanner (Axon Instruments, Foster City, CA). Images were analyzed using GenePix Pro 4.1 software (Axon Instruments). Any areas of the microarrays that had obvious blemishes were manually omitted from subsequent analyses. Spots were considered well measured only if the reference RNA fluorescence intensity was greater than 2.5 times the local background and if the regression correlation was greater than 0.6. Any clone that was not well measured on at least 70 % of the arrays was excluded from subsequent analysis. For each array, we used a scaling factor to set the mean sample/reference ratio for all well-measured spots to 1. For all subsequent analysis, log2 of this normalized sample/reference ratio was used. The data for each clone across all arrays within each of 2 array print runs were mean centered to minimize potential print run-specific bias. To analyze the data in an unsupervised manner, agglomerative hierarchial clustering was applied. In the supervised analysis of the data, the significance analysis of microarrays (SAM) was used.

Real-time quantitative polymerase chain reaction analysis (RQ-PCR) after reverse transcription was performed on selected genes. We used random hexamer priming and MuLV reverse transcriptase (fermentas, Hanover, MD) to generate cDNA. PCR was carried out on an Applied Biosystem Model 7700 Sequence Detector (Applied Biosystems, Darmstadt, Germany) using the QuantiTect SYBR Green PCR kit as described in the manufacturer's instructions (Qiagen AG, Hilden, Germany). The expression level of the SDHA gene was used to normalize for differences in input cDNA. Primer pairs used to measure mRNA abundances of the Interleukin-15 (IL-15) and SDHA genes were purchased from Qiagen AG (QuantiTect Primer Assays for human IL-15 and SDHA). IL-15 mRNA primers detected both IL-15 isoforms. Melting curve analyses were performed to verify the amplification specificity. Each sample was tested in duplicate. The expression ratio was calculated as 2n, where n was the CT value difference (IL-15 - SDHA) normalized by the CT difference of a calibrator sample.

The Mann-Whitney U test was applied to evaluate the significance of differences between sample mean values. Statistical significance was defined at P = 0.05.

### Example 1

Identification of differently expressed genes in patients with CNS3 compared to subjects with CNS1 status.

With the help of the gene expression measurements and analysis described above, patients with CNS3 status were compared to matched controls with CNS1 status (Table 1).

In the supervised analysis of the data, using SAM (1000 permutations; fold-change > 2; false discovery rate = 61 %), 18 differentially expressed genes were identified. To assure that differential gene expression was not simply a reflection of different percentages of normal bone marrow cells in the analytical specimens, purified leukemic cells from four precursor B-cell patients (two CNS3 and two CNS1 patients) with additional available kryopreserved diagnostic specimens were subjected to a second analysis. Out of the 18 genes, differential expression of IL-15 could be confirmed with an up to 10-fold higher expression in CNS-positive patients. IL-15 expression was validated by RQ-PCR in all patients included in the initial microarray analysis and found to be significantly different between CNS3 and CNS1 patients (Mann-Whitney U test P<0.001; Figure 1a). Furthermore, we were able to confirm the high expression of IL-15 in CNS3 patients in an independent set of 13 patients with CNS3 status compared to 26 CNS1 study subjects (Table 1) by RQ-PCR. Differential IL-15 expression was detected in a similar range as described above (Mann-Whitney U test, P< 0.001, Figure 1b). In a logistic regression analysis controlling gender, age and WBC at diagnosis, immunophenotype, and BCR/ABL, or TEL/AML1 fusion transcripts (yes/no), IL-15 expression levels above the median conferred a more than 10-fold increased risk of CNS3 status compared to expression equal to or below the median (odds ratio = 10.70, 95% Cl 2.95 - 38.81, P < 0.001). Thus, the expression of IL-15 is higher in leukemic cells from diagnostic bone marrow samples of ALL patients with overt CNS involvement.

### Example 2

Comparison of CNS-negative patients at initial diagnosis having CNS relapse with CNS-negative patients with no CNS relapse.

Next, it was analyzed if the expression level of IL-15 at initial diagnosis of CNS-negative patients could predict CNS relapse. The IL-15 expression was analyzed by RQ-PCR at initial diagnosis of ALL in 22 CNS1 patients subsequently relapsing with leukemic CNS involvement (nine isolated and 13 combined relapses) and 44 of the 52 patients without CNS disease at diagnosis also used in the two analyses above who remained in remission for more than three years (Table 2). A significantly higher IL-15 expression was detected in the diagnostic samples of patients with subsequent CNS relapse (Mann-Whitney, U test, P<0.001, Figure 1c) and in a logistic regression analysis controlling gender, age and WBC at diagnosis, immunophenotype, and BCR/ABL or TEL/AML1 fusion transcripts (yes/no), IL-15 expression levels above the median still conferred a more than 5-fold increased risk of CNS relapse compared to expression equal to or below the median (odds ratio = 5.39 %, Cl 1.37 - 21.23, P = 0.016). There was no significant difference in IL-15 expression levels detectable between isolated and combined relapses (Mann-Whitney U test, P = 0.764). Thus, higher expression levels of IL-15 in diagnostic leukemic bone marrow cells of patients with ALL and no overt CNS involvement are associated with CNS relapse.

Further date demonstrates that the same principle is given in AML and recurrent ALL.

**Table 1. Characteristics of 43 patients with acute lymphoblastic leukemia analyzed by gene expression profiling and 39 patients included in validation analysis stratified by CNS involvement at diagnosis.**

| | | Patients analyzed by gene expression profiling | | | Patients included in validation analysis | | |
|---|---|---|---|---|---|---|---|
| | | CNS3^{a} | CNS1^{b} | *P^{c}* | CNS3^{a} | CNS1^{b} | *P^{c}* |
| | | n (%) | n (%) | | n (%) | n (%) | |
| Age (years) | 1-<10 | 11(64.7) | 18 (69.2) | | 11 (84.6) | n (%) 13 (50.0) | |
| | ≥ 10 | 6 (35.3) | 8 (30.8) | 0.757 | 2 (15.4) | 13 (50.0) | 0.077 |
| | | | | | | | |
| Sex | male | 12 (70.6) | 18 (69.2) | | 5 (38.5) | 14 (58.2) | |
| | female | 5 (29.4) | 8 (30.8) | 0.925 | 8 (61.5) | 12 (41.8) | 0.365 |
| | | | | | | | |
| Presenting | < 10,000 | 3 (17.6) | 6 (23.1) | | 1 (7.7) | 1 (3.8) | |
| WBC^{d} count/µl | 10,000 - < 50,000 | 4 (23.5) | 7 (26.9) | | 6 (46.2) | 13 (59.0) | |
| | 50,000 - < 100,000 | 2 (11.8) | 3 (11.5) | | 3 (23.1) | 9 (34.6) | |
| | ≥ 100,000 | 8 (47.1) | 10 (38.5) | 0.946 | 3 (23.1) | 3 (11.5) | 0.705 |
| | | | | | | | |
| Immunopheno- | B-precursor | 9 (52.9) | 18 (69.2) | | 13 (100) | 26 (100) | |
| type | T-ALL | 8 (47.1) | 8 (30.8) | 0.280 | - | - | - |
| | | | | | | | |
| HCR/AHL positive | | - | - | - | 1 (7.7) | - | 0.333 |
| | | | | | | | |
| MLUAF4 positive | | - | - | - | - | - | - |
| | | | | | | | |
| TEL/AML1 positive | | 2 (11.8) | - | 0.151 | 2 (15.4) | - | 0.105 |

| | | | | | | | |
|---|---|---|---|---|---|---|---|
| ^{a} CNS3 (puncture nontraumatic, > 5 WBC/µL CSF with identifiable blasts); ^{b} CNS1 (puncture nontraumatic without leukemic blasts after cytocentrifugation); ^{c} *P* χ² or Fisher's exact test; ^{d} white blood cell. | | | | | | | |

**Table 2. Characteristics of 22 patients with acute lymphoblastic leukemia relapsing with CNS involvement and 44 patients being in long-term remission.**

| | | Relapse | Remission | |
|---|---|---|---|---|
| | | n (%) | n (%) | *P^{a}* |
| Age (years) | 1 - <10 | 15 (68.2) | 27 (61.4) | |
| | ≥ 10 | 7 (31.8) | 17 (38.6) | 0.587 |
| | | | | |
| Sex | male | 17 (77.3) | 26 (59.1) | |
| | female | 5 (22.7) | 18 (40.9) | 0.144 |
| | | | | |
| Presenting | < 10,000 | 6 (27.3) | 7 (15.9) | |
| WBC^{b} count/µl | 10,000 - < 50,000 | 4 (18.2) | 16 (36.4) | |
| | 50,000 - < 100,000 | 5 (22.7) | 9 (20.5) | |
| | ≥ 100,000 | 7 (31.8) | 12 (27.3) | 0.440 |
| | | | | |
| Immunopheno- | B-precursor | 19 (86.4) | 37 (84.1) | |
| type | T-ALL | 3 (13.6) | 7 (15.9) | 0.808 |
| | | | | |
| BCR/ABL positive | | 2 (9.1) | - | 0.108 |
| | | | | |
| MLL/AF4 positive | | - | - | - |
| | | | | |
| TEL/AML1 positive | | 2(9.1) | - | 0.108 |

| | | | | |
|---|---|---|---|---|
| ^{a} *P* χ² or Fisher's exact test, ^{b} white blood cell. | | | | |

## Claims

1. A method for the diagnosis of the involvement of the central nervous system in leukemia or lymphoma in a subject comprising
a) determining the relative level and/or absolute amount of interleukin-15 (IL-15) and/or lnterleukin-15 receptor alpha chain (IL-15Ralpha) in a sample from said subject
b) comparing the level and/or amount of IL-15 and/or IL-15Ralpha to the level and/or amount of IL-15 and/or IL-15Ralpha In a control sample, wherein the elevated level and/or amount of IL-15 and/or IL-15Ralpha in the sample from said subject relative to the control is indicative of the involvement of the central nervous system - a disease state where invasion of the meninges by leukemic cells is present - in leukemia or lymphoma in said subject.

2. A method for the stratification of a subject being afflicted with leukemia or lymphoma to determine the therapy regimen for the treatment of the leukemia or lymphoma comprising
a) determining the relative level and/or the absolute amount of IL-15 and/or IL-15Ralpha in a sample from said subject whereby the samples are obtained from bone marrow, blood, or cerebrospinal fluid;
b) comparing the level and/or amount of IL-15 and/or IL-15Ralpha to the level and/or amount of IL-16 and/or IL-15Ralpha in a control sample, with the level and/or amount of IL-15 and/or IL-15Ralpha In samples obtained prior to the begin of the therapy of said subject, or obtained in earlier stages of the regimen of said subject.

3. The method according to any one of the preceding claims wherein the level and/or amount of IL-15 and/or IL-15Ralpha is determined on mRNA level or on protein level.

4. The method according to any one of the preceding claims wherein the level of IL-15 (short and/or long form of IL-15) is determined.

5. The method according to any one of the preceding claims wherein the control sample is from an individual not afflicted with leukemia or lymphoma, or an individual afflicted with leukemia or lymphoma without involving the central nervous system - a disease state where invasion of the meninges by leukemic cells is present.

6. The method according to any one of the preceding claims wherein the elevated level is at least 2-fold, preferably at least 5-fold, relative to the level and/or amount of IL-15 and/or IL-15Ralpha of the control sample.

7. The method according to any one of the preceding claims wherein the subject is a human subject.

8. The method according to any one of claims 1 to 7. wherein the stratification is conducted based on the first diagnosis of leukemia or lymphoma with or without involvement of central nervous system - a disease state where invasion of the meninges by leukemic cells is present -, during initial treatment of leukemia or lymphoma with or without involvement of the central nervous system - a disease state where invasion of the meninges by leukemic cells is present -, after the first regimen of treatment of leukemia or lymphoma, or during monitoring of leukemia or lymphoma relapse.

9. The method according to claim 8 wherein the leukemia is childhood leukemia, preferably acute lymphoblastic leukemia or acute myelogenous leukemia.

## Patentansprüche

1. Verfahren zur Diagnose einer Involvierung des zentralen Nervensystems in Leukämie oder Lymphomerkrankungen in einem Individuum, umfassend
a) Bestimmen des relativen Niveaus und/oder der absoluten Menge an Interleukin-15 (IL-15) und/oder Interleukin-15 Rezeptor-alpha Kette (IL-15Ralpha) in einer Probe des Individuums, wobei die Proben aus Knochenmark, Blut oder cerebrospinaler Flüssigkeit stammen;
b) Vergleichen des Niveaus und/oder der Menge an IL-15 und/oder IL-15Ralpha gegenüber dem Niveau und/oder Menge an IL-15 und/oder IL-15Ralpha in einer Kontrollprobe, wobei ein erhöhtes Niveau und/oder Menge an IL-15 und/oder IL-15Ralpha in der Probe dieses Individuums relativ zu der Kontrolle ein Anzeichen für eine Involvierung des zentralen Nervensystems, einer Erkrankung, bei der eine Invasion der Hirnhaut durch Leukämiezellen auftritt, bei Leukämie oder Lymphomen in diesem Individuum ist.

2. Verfahren zur Stratifizierung eines Individuums, das an Leukämie oder Lymphom leidet, zur Bestimmung des Therapieplans oder Art zur Behandlung der Leukämie oder des Lymphoms umfassend
a) Bestimmung des relativen Niveaus und/oder der absoluten Menge an IL-15 und/oder IL-15Ralpha in einer Probe dieses Individuums, wobei die Proben aus Knochenmark, Blut oder cerebrospinaler Flüssigkeit stammen;
b) Vergleichen des Niveaus und/oder der Menge an IL-15 und/oder IL-15Ralpha mit dem Niveau und/oder der Menge an IL-15 und/oder IL-15Ralpha in einer Kontrollprobe, mit dem Niveau und/oder der Menge an IL-15 und/oder IL-15Ralpha in Proben erhalten vor dem Start der Therapie bei diesem Individuum oder Erhalten in früheren Stadien der Behandlung dieses Individuums.

3. Verfahren nach einem der vorherigen Ansprüche, wobei das Niveau und/oder die Menge an IL-15 und/oder IL-15Ralpha auf mRNA oder auf Proteinebene bestimmt wird.

4. Verfahren nach einem der vorherigen Ansprüche, wobei das Niveau an IL-15 (kurze und/oder lange Form von IL-15) bestimmt wird.

5. Verfahren nach einem vorherigen Ansprüche, wobei die Kontrollprobe von einem Individuum stammt, das nicht an Leukämie oder Lymphom leidet oder von einem Individuum, das an Leukämie oder Lymphom leidet, wobei dieses nicht das zentrale Nervensystem involviert, eine Erkrankung, bei der eine Invasion von Leukämiezellen in der Gehirnhaut vorliegt.

6. Verfahren nach einem der vorherigen Ansprüche, wobei das erhöhte Niveau mindestens zweifach, bevorzugt mindestens fünffach, relativ zu dem Niveau und/oder Menge an IL-15 und/oder IL-15Ralpha in der Kontrollprobe ist.

7. Verfahren nach einem der vorherigen Ansprüche, wobei das Individuum ein Mensch ist.

8. Verfahren nach einem der Ansprüche 1 bis 7, wobei die Stratifizierung durchgeführt wird basierend auf der ersten Diagnose von Leukämie oder Lymphom mit oder ohne Involvierung des zentralen Nervensystems, einem Krankheitszustand, bei dem eine Invasion von Leukämiezellen in der Gehirnhaut vorhanden ist, während der anfänglichen Behandlung der Leukämie oder des Lymphoms mit oder ohne Involvierung des zentralen Nervensystems, einer Erkrankung, bei der eine Invasion von Leukämiezellen in der Gehirnhaut vorliegt, nach der ersten Behandlung der Leukämie oder des Lymphoms oder bei der Überwachung eines möglichen Leukämie- oder Lymphomrückfalls.

9. Verfahren nach Anspruch 8, wobei die Leukämie eine Kinderleukämie ist, bevorzugt akute lymphoblastische Leukämie oder akute myeloide Leukämie.

## Revendications

1. Procédé pour le diagnostic de l'implication du système nerveux central dans une leucémie ou un lymphome chez un sujet comprenant
a) la détermination du niveau relatif et/ou de la quantité absolue d'interleukine-15 (IL-15) et/ou de chaîne alpha du récepteur de l'interleukine-15 (IL-15R-alpha) dans un échantillon provenant dudit sujet, les échantillons étant obtenus à partir de moelle osseuse, de sang ou de liquide céphalorachidien ;
b) la comparaison du niveau et/ou de la quantité d'IL-15 et/ou d'IL-15R-alpha au niveau et/ou à la quantité d'IL-15 et/ou d'IL-15R-alpha dans un échantillon témoin, dans lequel le niveau élevé et/ou la quantité élevée d'IL-15 et/ou d'IL-15Ralpha dans l'échantillon provenant dudit sujet par rapport au témoin témoigne de l'implication du système nerveux central - d'un état pathologique où l'invasion des méninges par des cellules leucémiques est présente - dans la leucémie ou le lymphome chez ledit sujet.

2. Procédé pour la stadification d'un sujet atteint d'une leucémie ou d'un lymphome pour déterminer le régime de thérapie pour le traitement de la leucémie ou du lymphome comprenant
a) la détermination du niveau relatif et/ou de la quantité absolue d'IL-15 et/ou d'IL-15R-alpha dans un échantillon provenant dudit sujet, les échantillons étant obtenus à partir de moelle osseuse, de sang ou de liquide céphalorachidien ;
b) la comparaison du niveau et/ou de la quantité d'IL-15 et/ou d'IL-15R-alpha au niveau et/ou à la quantité d'IL-15 et/ou d'IL-15R-alpha dans un échantillon témoin, avec le niveau et/ou la quantité d'IL-15 et/ou d'IL-15R-alpha dans des échantillons obtenus avant le début de la thérapie dudit sujet ou obtenus à des stades plus précoces du régime dudit sujet.

3. Procédé selon l'une quelconque des revendications précédentes dans lequel le niveau et/ou la quantité d'IL-15 et/ou d'IL-15R-alpha est déterminé d'après le niveau d'ARNm ou d'après le niveau de protéine.

4. Procédé selon l'une quelconque des revendications précédentes dans lequel le niveau d'IL-15 (forme courte et/ou longue d'IL-15) est déterminé.

5. Procédé selon l'une quelconque des revendications précédentes dans lequel l'échantillon témoin provient d'un individu non atteint de leucémie ou de lymphome ou d'un individu atteint de leucémie ou de lymphome sans implication du système nerveux central - un état pathologique où l'invasion des méninges par des cellules leucémiques est présente.

6. Procédé selon l'une quelconque des revendications précédentes dans lequel le niveau élevé est multiplié par au moins 2, de préférence multiplié par au moins 5, par rapport au niveau et/ou à la quantité d'IL-15 et/ou d'IL-15R-alpha de l'échantillon témoin.

7. Procédé selon l'une quelconque des revendications précédentes dans lequel le sujet est un sujet humain.

8. Procédé selon l'une quelconque des revendications 1 à 7, dans lequel la stadification est effectuée sur la base du premier diagnostic de leucémie ou de lymphome avec ou sans implication du système nerveux central - un état pathologique où l'invasion des méninges par des cellules leucémiques est présente -, pendant le traitement initial de la leucémie ou du lymphome avec ou sans implication du système nerveux central - un état pathologique où l'invasion des méninges par des cellules leucémiques est présente -, après le premier régime de traitement de la leucémie ou du lymphome ou pendant la surveillance de rechute de leucémie ou de lymphome.

9. Procédé selon la revendication 8 dans lequel la leucémie est une leucémie de l'enfant, de préférence la leucémie lymphoblastique aiguë ou la leucémie myéloïde aiguë.
